# FASCICULE DE BREVET EUROPEEN

(11) **EP 1 186 291 B1**
(45) Date de publication et mention de la délivrance du brevet: **02.07.2003**
(21) Numéro de dépôt: 01401890.7
(22) Date de dépôt: 13.07.2001
(51) Int. Cl.: A61K 7/48

(54) **Composition cosmétique, et son utilisation comme masque de nettoyage de la peau**
Kosmetische Zusammensetzung und deren Verwendung als Hautreinigungsmaske
Cosmetic composition, and the use thereof as a skin -cleansing mask

(30) Priorité: 07.09.2000 FR 0011409
(43) Date de publication de la demande: 13.03.2002
(73) Titulaire: L'OREAL, 75008 Paris (FR)
(72) Inventeur: Potin, Antony, 75006 Paris (FR); Touzan, Philippe, 75012 Paris (FR)
(74) Mandataire: Rasson, Catherine

(56) Documents cités:
- EP-A- 0 965 332
- US-A- 3 691 273
- US-A- 5 558 859
- US-A- 5 939 093

## Description

La présente invention se rapporte à une composition cosmétique contenant de l'eau, de l'alcool polyvinylique, un copolymère de vinylpyrrolidone et de l'huile, et à l'utilisation de ladite composition pour le nettoyage de la peau, et en particulier comme masque de nettoyage de la peau.

Les produits du type masque de beauté sont bien connus dans le domaine cosmétique. Ils se présentent notamment sous forme de gel, d'émulsion ou de pâte. Il est décrit dans la littérature, par exemple dans "Cosmetic and Toiletry Formulations", Seconde édition, Ernest W. Flick 1992, différentes formules de ce type de masque.

Ces masques de beauté peuvent être des masques hydratants sous forme de gel, qui n'ont pas vocation de nettoyage mais qui procurent à la peau un certain confort.

On connaît également des masques de nettoyage généralement sous forme d'émulsion, contenant en général une charge de type argileux. Toutefois, ces masques ont le désagrément de procurer à la peau une sensation de gras.

Enfin, il existe des masques s'enlevant par pelage, qui sont des compositions filmogènes aqueuses à base d'alcool polyvinylique. Ces masques de pelage (ou masque « peel-off »), après application sur le visage, sèchent pour donner un film que l'on retire par arrachage. Pendant le temps de séchage, l'effet occlusif du masque permet à la couche cornée (ou *stratum corneum*) de s'humidifier et de s'assouplir, ce qui peut favoriser la pénétration d'actif dans le cas où un ou des actifs sont inclus dans la composition. De plus, ces masques, lorsqu'ils sont retirés par arrachage, assurent un "peeling", entraînant notamment les cellules mortes des couches cornées de surface. Ils peuvent également éliminer les comédons et points noirs.

Toutefois, la qualité du pelage de ces masques est souvent décevante, notamment du fait que l'arrachage de ces masques s'avère généralement difficile car le film casse quand on le retire et l'arrachage doit donc être fait en plusieurs fois. Par ailleurs, le masque donne parfois l'impression d'être trop doux et il est alors ressenti comme inefficace, ou bien au contraire il peut faire mal quand on le retire, ce qui rend son utilisation rédhibitoire.

Il subsiste donc le besoin d'un masque pelable ne présentant pas les inconvénients de ceux de l'art antérieur.

La demanderesse a constaté de manière surprenante et inattendue qu'il était possible d'obtenir des compositions cosmétiques pouvant s'enlever par pelage et pouvant être utilisées comme masques de nettoyage de la peau, en associant à l'alcool polyvinylique, un copolymère de vinylpyrrolidone et de l'huile.

L'invention a donc pour objet une composition cosmétique comprenant de l'eau, de l'alcool polyvinylique, de 0.1% à 1.5% en poids par rapport au poids total de la composition d'un copolymère de vinylpyrrolidone et au moins une huile, l'alcool polyvinylique étant présent en une quantité allant de 5 à 25 % en poids par rapport au poids total de la composition.

La composition selon l'invention peut constituer notamment un masque qui a l'avantage de s'enlever facilement en une seule fois, après un temps de séchage d'environ 10 à 20 minutes, le temps de pose variant selon les conditions climatiques. Ce masque laisse la peau mate, douce et débarrassée des impuretés. En outre, il a l'avantage de présenter une stabilité améliorée par rapport aux compositions de l'art antérieur.

La composition selon l'invention est une composition cosmétique et, en tant que telle, contient un milieu physiologiquement acceptable, c'est-à-dire un milieu compatible avec la peau et/ou les muqueuses (lèvres).

Comme alcool polyvinylique, on peut utiliser dans la composition de l'invention, des polymères de différents degrés d'hydrolyse et/ou de différentes viscosités. On peut utiliser en particulier l'alcool polyvinylique ayant un degré d'hydrolyse variant de 74 à 99 % et/ou ayant une viscosité allant de 2,6 à 67 cps (2,6 à 67 mPa.s). Comme alcool polyvinylique, on peut citer par exemple les produits commercialisés sous les dénominations Airvol (Airvol 523, Airvol 540) par la société Air Products.

La quantité d'alcool polyvinylique dans la composition de l'invention va de 5 à 25 % en poids, et de préférence de 8 à 20 % en poids par rapport au poids total de la composition. Quand cette quantité est inférieure à 5 % en poids, le masque obtenu ne se pèle pas bien.

Dans la composition de l'invention, on peut utiliser tout type de copolymère de vinylpyrrolidone (PVP). On entend ici par copolymère aussi bien des polymères résultant de la polymérisation de la vinylpyrrolidone avec une seule sorte de monomère que ceux résultant de la polymérisation de la vinylpyrrolidone avec plusieurs sortes de monomères, et par exemple avec deux sortes de monomères conduisant à l'obtention de terpolymère.

Le copolymère de vinylpyrrolidone peut être notamment obtenu par polymérisation de la vinylpyrrolidone avec un ou plusieurs monomères choisis parmi les monomères acryliques ou méthacryliques, les monomères vinyliques, les hydrocarbures insaturés comme le styrène, le butadiène, l'hexadécène, l'eicosène, le décène, le triacontène. Comme indiqué ci-dessus, le copolymère avec la PVP peut être obtenu à partir de plusieurs de ces monomères.

Comme exemple de monomère acrylique ou méthacrylique, on peut citer l'acide acrylique, l'acide méthacrylique, et leurs esters comme l'acrylate d'éthyle, le méthacrylate d'éthyle et le méthacrylate de diméthylaminoéthyle.

Comme exemple de monomères vinyliques, on peut citer l'acétate de vinyle, le propionate de vinyle, le néodécanoate de vinyle, le pivalate de vinyle, le benzoate de vinyle, le t-butyl benzoate de vinyle et le vinylcaprolactame.

Comme copolymère particulièrement approprié pour la composition de l'invention, on peut citer par exemple les copolymères de vinylpyrrolidone et d'acétate de vinyle (nom CTFA : PVP/VA copolymer) comme les produits commercialisés sous la dénomination Luviskol VA 64 Poudre par la société BASF ou sous la dénomination PVPNA E335 par la société ISP ; les copolymères de vinylpyrrolidone et d'hexadécène (nom CTFA: PVP/hexadecene) comme le produit commercialisé sous la dénomination Antaron V-216 par la société ISP ; les copolymères de vinylpyrrolidone et de méthacrylate de diméthylaminoéthyle (nom CTFA : PVP/Dimethylaminoethylmethacrylate copolymer) (qui peuvent être aussi des terpolymères) comme les produits commercialisés sous les dénominations Copolymer 845, Copolymer 937 et Copolymer 958 par la société ISP ; les copolymères de vinylpyrrolidone et de triacontène (nom CTFA : Tricontanyl PVP) comme le produit commercialisé sous la dénomination Antaron WP-660 par la société ISP ; les copolymères de vinylpyrrolidone et d'eicosène (nom CTFA : PVP/Eicosene copolymer) comme le produit commercialisé sous la dénomination Antaron V-220 par la société ISP; les terpolymères de vinylpyrrolidone, d'acétate de vinyle et de propionate de vinyle (nom CTFA: PVP/VA/Vinylpropionate copolymer) comme les produits commercialisés sous les dénominations Luviskol VAP 3431 et Luviskol VAP 343 E par la société BASF ; les terpolymères de vinylpyrrolidone, vinylcaprolactame et méthacrylate de diméthylaminoéthyle (nom CTFA: Vinylcaprolactam/PVP/Dimethylaminoethyl-methacrylate copolymer) comme le produit commercialisé sous la dénomination Copolymer VC-713 par la société ISP, et leurs mélanges. Il est possible d'utiliser dans la composition de l'invention un copolymère de vinylpyrrolidone ou un mélange de tels copolymères.

La quantité de copolymère(s) de vinylpyrrolidone peut varier dans une large mesure. Toutefois, cette quantité ne doit pas être trop importante pour conserver les propriétés d'arrachage du masque et ne pas le rendre collant. Cette quantité est de 0,1 à 15 % en poids et de préférence de 1 à 10 % en poids par rapport au poids total de la composition.

La composition selon l'invention contient au moins une huile. Elle peut contenir aussi un mélange de plusieurs huiles. On peut utiliser tout huile habituellement utilisée dans les compositions cosmétiques, et par exemple les huiles d'origine végétale, telles que l'huile de noyaux d'abricot, le perhydrosqualène, l'huile d'avocat, l'huile de noix de macadamia, l'huile de tournesol, l'huile d'olive et l'huile de soja ; les huiles minérales comme l'huile de vaseline ; les huiles de synthèse comme le polyisobutène hydrogéné (huile de parléam), l'octyldodécanol, les esters d'acides gras et d'alcool gras (en C6-C30), les éthers d'alcool gras (en C4 à C30 saturés et/ou ramifiés) ; les huiles de silicone, notamment les huiles de silicone volatiles comme les cyclométhicones; les huiles fluorées, et leurs mélanges.

Selon un mode préféré de réalisation de l'invention, la ou les huiles sont choisies parmi les huiles d'origine végétale comme l'huile de noyaux d'abricot et le perhydrosqualène, les huiles de synthèse comme le polyisobutène hydrogéné et l'octyldodécanol, et leurs mélanges.

La quantité d'huiles peut varier dans une large mesure, et elle peut aller par exemple de 0,1 à 30 % en poids, de préférence de 1 à 20 % en poids et mieux de 1 à 10 % en poids par rapport au poids total de la composition.

La quantité d'eau dans la composition de l'invention peut représenter par exemple de 50 à 95 % en poids et de préférence de 55 à 90 % en poids par rapport au poids total de la composition.

La composition de l'invention peut contenir également des additifs habituels dans le domaine cosmétique, tels que les charges, les actifs, les conservateurs, les gélifiants, les plastifiants, les antioxydants, les solvants, les tensioactifs, les parfums, les absorbeurs d'odeur, les agents antimousse, des agents de douceur et les matières colorantes, dans la mesure où l'additif n'altère pas les propriétés recherchées pour la composition de l'invention. Les quantités de ces différents additifs sont celles classiquement utilisées dans les domaines considérés, et par exemple de 0,01 à 25 % du poids total de la composition.

Les agents de douceur apportent de la douceur lors de l'arrachage du masque. L'agent de douceur peut être choisi par exemple parmi les polydiméthylsiloxanes oxyéthylénés, le diméthylisosorbide ou leurs mélanges Selon un mode particulier de réalisation de l'invention, la composition contient au moins un agent de douceur.

Comme gélifiants, on peut citer en particulier les gélifiants hydrophiles tels que :
- les dérivés cellulosiques (carboxyméthylcellulose, hydroxyéthylcellulose, hydroxypropylméthylcellulose) ;
- les gommes naturelles telles que les gommes de xanthane, de guar, de caroube, les carraghénanes ;
- les polymères polycarboxyvinyliques du type Carbomer, tels que ceux vendus par la société Goodrich sous les dénominations Carbopol 940, 951, 980, ou par la société 3V-Sigma sous la dénomination Synthalen K ou Synthalen L ;
- les copolymères acryliques tels que les copolymères d'acrylates/alkylacrylates vendus sous les dénominations Pemulen par la société Goodrich ;
- les polyacrylamides et les copolymères d'acrylamide, tels que le produit vendu sous le nom de SEPIGEL 305 par la société SEPPIC (nom CTFA : polyacrylamide/C13-14 isoparaffin/Laureth-7) et le produit vendu sous le nom de HOSTACERIN AMPS par la société HOECHST (nom CTFA : Ammonium polyacryldimethyltauramide) ;
- les polymères associatifs tels que notamment les polyuréthannes associatifs.

Selon un mode préféré de réalisation de l'invention, la composition contient au moins un solvant. Comme solvants ou agents de coalescence, on peut citer par exemple les alcools inférieurs c'est-à-dire comportant de 1 à 4 atomes de carbone, tels que l'éthanol, les polyols et leurs dérivés tels que le propylène glycol, le dipropylène glycol, le butylène-1,3 glycol, la glycérine, la polyglycérine, le sorbitol, le diméthylisosorbide, et leurs mélanges. La quantité de solvant(s) dans la composition selon l'invention peut aller par exemple de 1 à 25 % et mieux de 5 à 20 % en poids par rapport au poids total de la composition.

Comme tensioactifs, on peut utiliser par exemple les tensioactifs non ioniques et notamment les polymères d'oxyde d'éthylène (PEG) tels que le polyéthylène glycol 4000 (nom CTFA : PEG-75). La quantité de tensioactif dans la composition selon l'invention peut aller par exemple de 0,1 à 10 % en poids et mieux de 0,5 à 5 % en poids par rapport au poids total de la composition.

La composition de l'invention peut contenir un actif habituellement utilisé dans le domaine cosmétique. Comme actifs utilisables dans la composition de l'invention, on peut citer plus particulièrement les α-hydroxy-acides comme les acides glycolique, lactique, malique, tartrique, citrique, mandélique ; les β-hydroxy-acides comme l'acide salicylique ainsi que ses dérivés acylés, les acides hydroxy-2 alcanoïques et leurs dérivés, et les composés décrits dans les documents FR-A-2581542 et EP-A-378936, et notamment les acides n-octanoyl-5-salicylique (ou capryloyl salicylique), n-décanoyl-5-salicylique, n-dodécanoyl-5-salicylique, n-octyl-5-salicylique, n-heptyloxy-5-salicylique, n-heptyloxy-4-salicylique ; les extraits végétaux comme l'extrait hydroalcoolique de racines de mûrier (Mulberry extract BG de la société Maruzen) et leurs mélanges.

Ainsi qu'indiqué ci-dessus, la composition cosmétique de l'invention peut constituer notamment un produit de nettoyage de la peau, plus particulièrement sous forme d'un masque destiné aux soins du visage, d'une partie du visage ou du cou, afin notamment de nettoyer la peau en profondeur, par exemple par élimination des cellules mortes de la couche cornée de surface ou par élimination des corps gras présents en excès à la surface de la peau (le sébum par exemple), de raffermir la peau, de l'adoucir et/ou de préparer la peau à subir un traitement ultérieur particulier.

L'invention a aussi pour objet l'utilisation cosmétique d'une composition telle que décrite ci-dessus, comme masque de nettoyage de la peau.

L'invention a également pour objet un procédé de traitement cosmétique pour le nettoyage de la peau, caractérisé par le fait que l'on applique sur la peau, une composition telle que décrite ci-dessus.

Les exemples ci-après de compositions selon l'invention sont donnés à titre d'illustration et sans caractère limitatif. Les quantités y sont données en % en poids, sauf mention contraire.

### Exemple 1

### Phase A

- PEG-75 1 %
- Conservateurs 0,2 %
- Eau 59,8 %

### Phase B

- Alcool polyvinylique 11 %

### Phase C

- Diméthylisosorbide 1,5 %
- Polydiméthylsiloxane oxyéthyléné (Dow Corning 193 Surfactant de la société Dow Corning) 2 %

### Phase D

- Glycérine 2 %
- Gomme de xanthane 0,5 %

### Phase E

- Ethanol 12,5 %
- Acide capryloyl salicylique 0,5 %
- Extrait hydroalcoolique de racines de mûrier (à 1% de MA) 2 %

### Phase F

- Huile de parléam 3 %
- PVP/VA copolymer 4 %

Mode opératoire : On chauffe la phase A sous agitation à 90°C jusqu'à solubilisation. On y ajoute la phase B et on agite jusqu'à homogénéisation. On abaisse la température du mélange à 70°C avant d'y ajouter sous agitation la phase C. Puis, on abaisse la température du mélange à 60°C avant d'y introduire sous agitation la phase D préalablement préparée par dispersion de la gomme de xanthane dans la glycérine. On abaisse ensuite la température du mélange à 25°C puis on y introduit les phases E et F successivement sous agitation.

La composition obtenue s'applique comme un masque et s'élimine par arrachage après un certain temps de pause (environ 10 minutes) en laissant une peau douce et nettoyée.

### Test de pelage

Un panel d'une trentaine de consommateurs a testé le masque de l'exemple 1 selon l'invention et des masques constituant des exemples comparatifs :

| Composition | Exemple 1 de l'invention | Exemple comparatif 1 | Exemple comparatif 2 | Exemple comparatif 3 |
|---|---|---|---|---|
| PEG-75 | 1% | 1% | 1% | 1% |
| Alcool polyvinylique | 11% | 11% | 11% | 11% |
| Diméthylisosorbide | 1,5% | 1,5% | 1,5% | 1,5% |
| Polydiméthylsiloxane oxyéthyléné | 2% | 2% | 2% | 2% |
| Glycérine | 2% | 2% | 2% | 2% |
| Gomme de xanthane | 0,5% | 0,5% | 0,5% | 0,5% |
| Ethanol | 12,5% | 12,5% | 12,5% | 12,5% |
| Actifs | 2,5% | 2,5% | 2,5% | 2,5% |
| Huile de parléam | 3% | - | 3% | - |
| PVP/VA Copolymer | 4% | - | - | 4% |
| Eau | qsp 100% | qsp 100% | qsp 100% | qsp 100% |

La qualité du pelage a été évaluée sur une échelle de 1 à 15. Le tableau ci-dessous donne les résultats.

| | Moyenne des évaluations |
|---|---|
| Exemple 1 selon l'invention | 9,4 |
| Exemple comparatif 1 | 7,3 |
| Exemple comparatif 2 | 8,0 |
| Exemple comparatif 3 | 8,2 |

Ce tableau montre que le masque obtenu selon l'invention est plus facilement pelable que ceux des exemples comparatifs.

### Exemple 2 :

### Phase A

- PEG-75 1 %
- Conservateurs 0,2 %
- Eau 63,1 %

### Phase B

- Alcool polyvinylique 11 %

### Phase C

- Polydiméthylsiloxane oxyéthyléné 2 %

### Phase D

- Glycérine 2,7 %
- Gomme de xanthane 0,5 %

### Phase E

- Ethanol 12,5 %

### Phase F

- PVP/VA copolymer 4 %
- Huile de parléam 3 %

Le mode opératoire est le même que pour l'exemple 1. On obtient un masque qui s'arrache facilement et laisse la peau bien nettoyée.

## Revendications

1. Composition cosmétique comprenant de l'eau, de l'alcool polyvinylique, au moins un copolymère de vinylpyrrolidone et au moins une huile, l'alcool polyvinylique étant présent en une quantité allant de 5 à 25 % en poids par rapport au poids total de la composition et la quantité de copolymère allant de 0,1 à 15 % en poids par rapport au poids total de la composition.

2. Composition selon la revendication 1, **caractérisée par le fait que** le copolymère de vinylpyrrolidone est obtenu par polymérisation de la vinylpyrrolidone avec un ou plusieurs monomères choisis parmi les monomères acryliques ou méthacryliques, les monomères vinyliques, les hydrocarbures insaturés.

3. Composition selon la revendication précédente, **caractérisée par le fait que** l'hydrocarbure insaturé est choisi parmi le styrène, le butadiène, l'hexadécène, l'eicosène, le décène, le triacontène et leurs mélanges.

4. Composition selon l'une quelconque des revendications précédentes, **caractérisés par le fait que** le copolymère de vinylpyrrolidone est choisi parmi les copolymères de vinylpyrrolidone et d'acétate de vinyle, les copolymères de vinylpyrrolidone et d'hexadécène, les copolymères de vinylpyrrolidone et de méthacrylate de diméthylaminoéthyle, les copolymères de vinylpyrrolidone et de triacontène, les copolymères de vinylpyrrolidone et d'eicosène, les terpolymères de vinylpyrrolidone, d'acétate de vinyle et de propionate de vinyle, les terpolymères de vinylpyrrolidone, vinylcaprolactame et méthacrylate de diméthylaminoéthyle, et leurs mélanges.

5. Composition selon l'une quelconque des revendications précédentes, **caractérisée par le fait que** l'huile est choisie parmi les huiles d'origine végétale, les huiles de synthèse et leurs mélanges.

6. Composition selon l'une quelconque des revendications précédentes, **caractérisée par le fait que** la quantité d'huile(s) va de 0,1 à 30 % en poids par rapport au poids total de la composition.

7. Composition selon l'une quelconque des revendications précédentes, **caractérisée par le fait que** la quantité d'eau va de 50 à 95 % en poids par rapport au poids total de la composition.

8. Composition selon l'une quelconque des revendications précédentes, **caractérisée par le fait qu'**elle comprend en outre un solvant choisi parmi les alcools comportant de 1 à 4 atomes de carbone, les polyols et leurs dérivés, et leurs mélanges.

9. Composition selon l'une quelconque des revendications précédentes, **caractérisée par le fait qu'**elle comprend en outre un agent de douceur choisi parmi les polydiméthylsiloxanes oxyéthylénés, le diméthylisosorbide ou leurs mélanges.

10. Composition selon l'une quelconque des revendications précédentes, **caractérisée par le fait qu'**elle comprend en outre un actif choisi parmi les α-hydroxy-acides, les β-hydroxy-acides, les extraits végétaux et leurs mélanges.

11. Composition selon l'une quelconque des revendications précédentes, **caractérisée par le fait qu'**elle constitue un produit de nettoyage de la peau.

12. Composition selon l'une quelconque des revendications 1 à 10, **caractérisée par le fait qu'**elle constitue un masque.

13. Composition selon la revendication précédente, **caractérisée par le fait qu'**elle constitue un masque peel-off.

14. Utilisation cosmétique de la composition selon l'une quelconque des revendications 1 à 10, comme masque de nettoyage de la peau.

15. Procédé de traitement cosmétique pour le nettoyage de la peau, **caractérisé par le fait que** l'on applique sur la peau, une composition selon l'une quelconque des revendications 1 à 10.

## Patentansprüche

1. Kosmetische Zusammensetzung, die Wasser, Polyvinylalkohol, mindestens ein Vinylpyrrolidon-Copolymer und mindestens ein Öl enthält, wobei der Polyvinylalkohol in einer Menge von 5 bis 25 Gew.-%, bezogen auf das Gesamtgewicht der Zusammensetzung, enthalten ist und der Mengenanteil des Vinylpyrrolidon-Copolymers im Bereich von 0,1 bis 15 Gew.-%, bezogen auf das Gesamtgewicht der Zusammensetzung, liegt.

2. Zusammensetzung nach Anspruch 1, **dadurch gekennzeichnet, dass** das Vinylpyrrolidon-Copolymer durch Polymerisation von Vinylpyrrolidon mit einem oder mehreren Monomeren hergestellt wird, die unter den Acrylmonomeren, Methacrylmonomeren, Vinylmonomeren und ungesättigten Kohlenwasserstoffen ausgewählt sind.

3. Zusammensetzung nach dem vorhergehenden Anspruch, **dadurch gekennzeichnet, dass** der ungesättigte Kohlenwasserstoff unter Styrol, Butadien, Hexadecen, Eicosen, Decen, Triaconten und deren Gemischen ausgewählt ist.

4. Zusammensetzung nach einem der vorhergehenden Ansprüche, **dadurch gekennzeichnet, dass** das Vinylpyrrolidon-Copolymer,unter den Copolymeren von Vinylpyrrolidon und Vinylacetat, den Copolymeren von Vinylpyrrolidon und Hexadecen, den Copolymeren von Vinylpyrrolidon und Dimethylaminoethylmethacrylat, den Copolymeren von Vinylpyrrolidon und Triaconten, den Copolymeren von Vinylpyrrolidon und Eicosen, den Terpolymeren von Vinylpyrrolidon, Vinylacetat und Vinylpropionat, den Terpolymeren von Vinylpyrrolidon, Vinylcaprolactam und Dimethylaminoethylmethacrylat und deren Gemischen ausgewählt ist

5. Zusammensetzung nach einem der vorhergehenden Ansprüche, **dadurch gekennzeichnet, dass** das Öl unter den Ölen pflanzlicher Herkunft, den synthetischen Ölen und deren Gemischen ausgewählt ist.

6. Zusammensetzung nach einem der vorhergehenden Ansprüche, **dadurch gekennzeichnet, dass** der Mengenanteil des Öls oder der Öle im Bereich von 0,1 bis 30 Gew.-%, bezogen auf das Gesamtgewicht der Zusammensetzung, liegt.

7. Zusammensetzung nach einem der vorhergehenden Ansprüche, **dadurch gekennzeichnet, dass** der Mengenanteil des Wassers im Bereich von 50 bis 95 Gew.-%, bezogen auf das Gesamtgewicht der Zusammensetzung, liegt.

8. Zusammensetzung nach einem der vorhergehenden Ansprüche, **dadurch gekennzeichnet, dass** sie ferner ein Lösungsmittel enthält, das unter den Alkoholen mit 1 bis 4 Kohlenstoffatomen, den Polyolen und ihren Derivaten und deren Gemischen ausgewählt ist.

9. Zusammensetzung nach einem der vorhergehenden Ansprüche, **dadurch gekennzeichnet, dass** sie ferner ein Mittel für die Geschmeidigkeit enthält, das unter den ethoxylierten Polydimethylsiloxanen, Dimethylisosorbid oder deren Gemischen ausgewählt ist.

10. Zusammensetzung nach einem der vorhergehenden Ansprüche, **dadurch gekennzeichnet, dass** sie ferner einen Wirkstoff enthält, der unter den α-Hydroxysäuren, den β-Hydroxysäuren, den Pflanzenextrakten und deren Gemischen ausgewählt ist.

11. Zusammensetzung nach einem der vorhergehenden Ansprüche, **dadurch gekennzeichnet, dass** es sich um ein Produkt zur Reinigung der Haut handelt.

12. Zusammensetzung nach einem der Ansprüche 1 bis 10, **dadurch gekennzeichnet, dass** es sich um eine Maske handelt.

13. Zusammensetzung nach dem vorhergehenden Anspruch, **dadurch gekennzeichnet, dass** sie eine Peel-off-Maske ist.

14. Kosmetische Verwendung der Zusammensetzung nach einem der Ansprüche 1 bis 10 als Reinigungsmaske für die Haut.

15. Verfahren zur kosmetischen Reinigung der Haut, **dadurch gekennzeichnet, dass** auf die Haut eine Zusammensetzung nach einem der Ansprüche 1 bis 10 aufgebracht wird.

## Claims

1. Cosmetic composition comprising water, polyvinyl alcohol, at least one copolymer of vinylpyrrolidone and at least one oil, the polyvinyl alcohol being present in a quantity ranging from 5 to 25% by weight relative to the total weight of the composition and the quantity of copolymer ranging from 0.1 to 15% by weight relative to the total weight of the composition.

2. Composition according to Claim 1, **characterized in that** the copolymer of vinylpyrrolidone is obtained by polymerization of vinylpyrrolidone with one or more monomers chosen from acrylic or methacrylic monomers, vinyl monomers and unsaturated hydrocarbons.

3. Composition according to the preceding claim, **characterized in that** the unsaturated hydrocarbon is chosen from styrene, butadiene, hexadecene, eicosene, decene, triacontene and mixtures thereof.

4. Composition according to any one of the preceding claims, **characterized in that** the copolymer of vinylpyrrolidone is chosen from copolymers of vinylpyrrolidone and vinyl acetate, copolymers of vinylpyrrolidone and hexadecene, copolymers of vinylpyrrolidone and dimethylaminoethyl methacrylate, copolymers of vinylpyrrolidone and triacontene, copolymers of vinylpyrrolidone and eicosene, terpolymers of vinylpyrrolidone, vinyl acetate and vinyl propionate, terpolymers of vinylpyrrolidone, vinylcaprolactam and dimethylaminoethyl methacrylate, and mixtures thereof.

5. Composition according to any one of the preceding claims, **characterized in that** the oil is chosen from oils of plant origin, synthetic oils and mixtures thereof.

6. Composition according to any one of the preceding claims, **characterized in that** the quantity of oil(s) ranges from 0.1 to 30% by weight relative to the total weight of the composition.

7. Composition according to any one of the preceding claims, **characterized in that** the quantity of water ranges from 50 to 95% by weight relative to the total weight of the composition.

8. Composition according to any one of the preceding claims, **characterized in that** it additionally comprises a solvent chosen from alcohols comprising 1 to 4 carbon atoms, polyols and derivatives thereof, and mixtures thereof.

9. Composition according to any one of the preceding claims, **characterized in that** it additionally comprises an agent for imparting softness, chosen from oxyethylenated polydimethylsiloxanes, dimethylisosorbide and mixtures thereof.

10. Composition according to any one of the preceding claims, **characterized in that** it additionally comprises an active agent chosen from α-hydroxy acids, β-hydroxy acids, plant extracts and mixtures thereof.

11. Composition according to any one of the preceding claims, **characterized in that** it constitutes a skin cleansing product.

12. Composition according to any one of Claims 1 to 10, **characterized in that** it constitutes a mask.

13. Composition according to the preceding claim, **characterized in that** it constitutes a peel-off mask.

14. Cosmetic use of the composition according to any one of Claims 1 to 10, as a skin cleansing mask.

15. Method of cosmetic treatment for cleansing the skin, **characterized in that** a composition according to any one of Claims 1 to 10 is applied to the skin.
